# EUROPEAN PATENT SPECIFICATION

(11) **EP 0 425 448 B1**
(45) Date of publication and mention of the grant of the patent: **25.01.1995**
(21) Application number: 90830421.5
(22) Date of filing: 25.09.1990
(51) Int. Cl.: A61M 5/32

(54) **Safety cap for a medical needle after use**
Sicherheitskappe für eine gebrauchte medizinische Nadel
Capuchon de sécurité pour une aiguille médicale usagée

(30) Priority: 26.09.1989 IT 1961889 U
(43) Date of publication of application: 02.05.1991
(73) Proprietor: Azzali, Luigi, I-46017 Rivarolo Mantovano (MN) (IT)
(72) Inventor: Azzali, Luigi, I-46017 Rivarolo Mantovano (MN) (IT)
(74) Representative: Modiano, Guido, Dr.-Ing.

(56) References cited:
- EP-A- 0 265 159
- EP-A- 0 364 839
- WO-A-90/06145

## Description

The design is a safety cap for a medical needle after its use.

It is well known that a large number of therapeutical treatments implies the connection of the patient to a line which conveys a fluid, such as an extracorporeal line or even a simple line to introduce a medicament.

The connection of the line to the patient is carried out by a device called butterfly needle which includes, at the end of the line, the injection needle as such and two flexible wings - usually made of a plastic material - used to facilitate the fixing of the set to the patient.

Such butterfly needle can be of varios dimensions, and when it is joined to a fistula with which the patient is supplied, it is also called fistula needle.

It is also known that injections to patients are nowadays mainly done-both in hospital and at home - by means of disposable syringes which are disposed of together with the needle, when the operator has completed the injection.

Ever since it leaves the place of production and until its use, the needle on the butterfly and the needle on the dispotsable syringe is inserted in a protective cap which is taken away by the operetor when the needle must be inserted in the body of the patient.

EP-A-265 159 discloses an open sheathlike protector device for butterfly needles provided with two elastically deformable extensions defining an opening to house the butterfly.

It is obvious that after its use the needle, which is now infected, has to be protected again to allow its disposal without any risk of contamination and here the use of the protective cap suitable for the new needle could create some problems.

The first problem is the difficulty to insert the needle in the cap, which has a reduced feeding aperture: a wrong insertion is then most likely, and this would have as a consequence that the needle could prick the operator, and this must absolutely be avoided.

The second problem consists in the fact that the connection of the cap to the needle is very precarius; the needle undergoes much handling from the moment it ceases to be used to the moment it is finally eliminated in suitable containers, and the cap can then slip off the needle, which can now pierce such containers and come in contact with the worker.

US-A-4747835 discloses a safety cap for medical needles having means to lock such needles in the cap.

It is the purpose of the present design to create a safety cap for a medical needle, to be used after such needle has been employed, which would first of all allow an easy insertion of the needle, and then would assure the locking of the needle once it is in, so as to eliminate any possibility of contamination due to the contact of an infected needle with the operator.

Such task is fulfilled by a safety cap for used medical needles, according to the design, as defined in the appended claim 1.

Further characteristics and advantages will mainly stand out from the description of one form of execution, which can be preferred but not exclusive, of the design and shown to illustrate and not to limit the enclosed drawings, where:
- picture 1 shows the design in a perspective view according to a first form of realization, with a butterfly needle ready for insertion; and
- picture 2 represents a section with the plane II - II of picture 1 of the design-a continuous line being the needle waiting to be inserted, and a dot-dash line being the inserted needle.

With reference to the above pictures 1 and 2, globally indicated by 1, is the safety cap according to the disegn for the butterfly needle- globally indicated by 2- which as known includes the needle as such 2a and the butterfly 2b, normally made of plastic, set at the end of a cannula, 2c.

The safety cap 1 comprises an elongated hollow body 3, closed at one end and having at the other end the two opposite extension 4 and 5, made of resiliant material, that are shaped so as to define the portion of space 6 suitable for the housing of the butterfly 2b, and which have along the edge of such potion of space indents as in 7 for extension 4, and 8 for extension 5, which have the function of holding the butterfly 2b in its housing 6, once it has been introduced thanks to the possibility of elastic deformation of the material of the extentions 4 and 5.

It is also interesting to point out how the edeges of the elastic extentions 4 and 5 are off the longitudinal axle of the cap in the part incuded between the free extremity and the indents to hold the butterfly, as for instance 9 for extention 5; this is to determine an appropriate facilitation for the insertion of the butterfly itself.

The functioning of the design is evident.

The operator holds in one hand cap 1 and in the other hand the butterfly needle 2, taking this to the opening of the cap, which presents the elastic extensions 4 and 5 in an undeformed position - as shown in picture 1 and with a solid line in picture 2.

The operator now inserts the butterfly needle in the cap - and he can do so with great ease thanks to the relevant opening assured by the feeding edges as in 9 - and he pushes it in to the point where the extentions 4 and 5 begin to deform elastically, thus allowing the isertion of butterfly 2b in the portion of space 6, as shouwn in dash-and-dot in picture 2, with a springing back to the undeformed shape of extensions 4 and 5, and consequent locking done by the indents as 7 and 8 of butterfly 2b: thus the butterfly needle is definitily imprisoned in its cap , in a virtually irreversable situation.

From the above it is understandable how the aim of the safety cap according to the design has been perfectly reached, as it excludes that an infected needle might come into contact with whomsoever, both during the insertion of the cap and after the insertion is completed.

The described design is susceptible to a number of modifications and variants, all falling within the inventive concept; furthermore all the details can be substituted with other technically equivalent elements.

In the pratical realization of the design the materials employed as well as shapes and dimensions can vary according to the need.

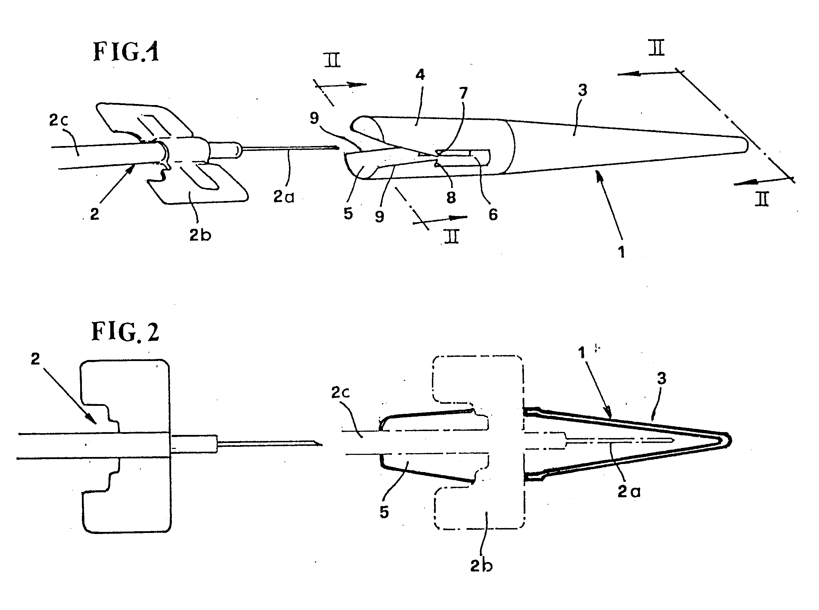

## Claims

1. Safety cap (1) for a butterfly/fistula needle (2) after its use, the safety cap comprising an elongated hollow body (3) closed at one end and having at the other end two opposite elastically deformable extensions (4,5) defining an opening (6) to house the butterfly (2b), said extensions having indents (7,8) at the introduction side of the opening to hold the butterfly (2b) after its introduction in said opening through elastic deformation of the extensions (4,5).

2. The safety cap of claim 1, characterised in that the edges of the opposite extensions have, in the part included between the free end and the indents (4,5) to hold the butterfly, an inclination (9) from the longitudinal axis of the cap such as to determine an effective feeding aperture for the insertion of the butterfly (2b).

## Patentansprüche

1. Sicherheitskappe (1) für eine gebrauchte medizinische Nadel (2), insbesondere eine Butterfly-Nadel, mit einem Hohlkörper (3), der an einem Ende geschlossen ist und der am anderen Ende zwei einander gegenüberliegende elastisch verformbare Fortsätze (4; 5) aufweist, die eine Ausnehmung (6) für die Aufnahme des Flügelteils (2b) der Nadel (2) begrenzen, wobei diese Fortsätze (4; 5) an der Einführseite der Ausnehmung (6) Absätze (6; 7) aufweisen, mittels der im Anschluß an eine elastische Verformung der Fortsätze (4; 5) der Flügelteil (2b) nach dem Einführen in der Ausnehmung (6) vorriegelbar ist.

2. Sicherheitskappe nach Anspruch 1,
dadurch gekennzeichnet, daß die Ränder (9) der einander gegenüberliegenden Fortsätze (4; 5) in dem zwischen dem freien Ende und der Ausnehmung (6) gelegenen Abschnitt gegenüber dar Längsachse der Sicherheitskappe (1) geneigt verlaufen und dadurch eine Führung beim Einführen der Flügel (2b) bilden.

## Revendications

1. Capuchon de sécurité (1) destiné à une aiguille papillon/à fistule (2) usagée, le capuchon de sécurité comprenant un corps creux allongé (3) fermé à une extrémité et présentant à l'autre extrémité deux prolongements (4, 5) opposés déformables élastiquement délimitant une ouverture (6) destinée à loger le papillon (2b), ces prolongements possédant des entailles (7, 8) du côté d'introduction de l'ouverture afin de maintenir le papillon (2b) après son introduction dans ladite ouverture par déformation élastique des prolongements (4, 5).

2. Capuchon de sécurité selon la revendication 1, caractérisé en ce que les bords des prolongements opposés possèdent, dans la partie comprise entre l'extrémité libre et les entailles (4, 5) destinées à maintenir le papillon, une inclinaison (9) par rapport à l'axe longitudinal du capuchon telle qu'elle constitue une ouverture d'introduction adéquate pour l'insertion du papillon (2b).
